# EUROPEAN PATENT SPECIFICATION

(11) **EP 3 225 610 B1**
(45) Date of publication and mention of the grant of the patent: **29.09.2021**
(21) Application number: 15863828.8
(22) Date of filing: 05.11.2015
(51) Int. Cl.: C07B 37/04, C07C 1/32, C07C 13/567, C07C 41/30, C07C 43/205, C07C 209/68, C07C 211/56, C08G 61/12

(54) **PROCESS FOR THE PREPARATION OF POLYMERS BY COUPLING REACTIONS**
VERFAHREN ZUR HERSTELLUNG VON POLYMEREN MITTELS KUPPLUNGSREAKTIONEN
PROCÉDÉ DE FABRICATION DES POLYMÈRES PAR RÉACTIONS DE COUPLAGE

(30) Priority: 26.11.2014 JP 2014238557
(43) Date of publication of application: 04.10.2017
(73) Proprietor: SUMITOMO CHEMICAL COMPANY, LIMITED, Chuo-ku Tokyo 104-8260 (JP)
(72) Inventor: YOKOI, Yuki, Tsukuba-shi Ibaraki 300-3294 (JP); KASHIMA, Ken, Osaka-shi Osaka 554-8558 (JP)
(74) Representative: Winter, Brandl - Partnerschaft mbB
(86) International application number: PCT/JP2015/081107
(87) International publication number: WO 2016/084565

(56) References cited:
- WO-A1-00/53656
- WO-A1-97/05104
- WO-A1-98/40331
- WO-A1-2004/106351
- WO-A1-2009/054832
- WO-A1-2012/133874
- WO-A1-2012/133875
- WO-A1-2014/007404
- WO-A2-2005/058916
- WO-A2-2008/080992
- JP-A- H08 501 107
- JP-A- H11 509 856
- JP-A- 2000 336 045
- US-A1- 2002 016 512
- US-B1- 6 627 764
- SHARMA, N. ET AL.: "Pd-Catalyzed Orthogonal Knoevenagel/Perkin Condensation-Decarboxylation-Heck/Suzuki Sequences: Tandem Transformations of Benzaldehydes into Hydroxy-Functionalized Antidiabetic Stilbene-Cinnamoyl Hybrids and Asymmetric Distyrylbenzenes", CHEMISTRY - A EUROPEAN JOURNAL, vol. 17, no. 37, 2011, pages 10350-10356, XP55484619, ISSN: 0947-6539, DOI: 10.1002/chem.201101174
- NIFANT'EV, I. E ET AL.: "Asymmetric ansa-Zirconocenes Containing a 2-Methyl-4-aryltetrahydroindacene Fragment: Synthesis, Structure, and Catalytic Activity in Propylene Polymerization and Copolymerization", ORGANOMETALLICS, vol. 30, no. 21, 2011, pages 5744-5752, XP008162994, ISSN: 0276-7333, DOI: 10.1021/OM200610B
- YILMAZ, ULKU ET AL.: 'Synthesis of novel benzimidazole salts and microwave-assisted catalytic activity of in situ generated Pd nanoparticles from a catalyst system consisting of benzimidazol salt, Pd(OAc)2, and base in a Suzuki-Miyaura reaction' TURKISH JOURNAL OF CHEMISTRY vol. 37, no. 5, 01 January 2013, pages 721 - 733, XP055448001 DOI: 10.3906/KIM-1207-18
- MONOPOLI, ANTONIO ET AL.: 'Palladium/zirconium oxide nanocomposite as a highly recyclable catalyst for C-C coupling reactions in water' MOLECULES vol. 15, 24 June 2010, pages 4511 - 4525, XP055448003 DOI: 10.3390/MOLECULES15074511
- None

## Description

### Technical Field

The present invention relates to a production method of a compound.

### Background Art

A compound production method using a coupling reaction of an organic boron compound and an organic halogen compound is a production method having higher general versatility. Thus, organic semiconductor materials (for example, organic electroluminescent materials, organic transistor materials and organic solar battery materials), medicinal materials, agrichemical materials, liquid crystal materials, or the like are produced using the coupling reaction.

Patent documents 1 and 2 describe a compound production method comprising a step of coupling-reacting an organic boron compound with an organic halogen compound in the presence of a transition metal catalyst, an organic base and an organic solvent. Patent documents 1 and 2 do not describe a compound production method using a compound having two or more hydroxyl groups. Patent document 3 describes a compound production method comprising a step of coupling-reacting an organic boron compound with an organic halogen compound in the presence of a transition metal catalyst, an inorganic base, two or more hydroxyl groups and an organic solvent. Patent document 3 does not describe a compound production method using an organic base.

### Prior Art Document

### Patent Document

Patent document 1: Japanese Patent Application National Publication No. 2002-539287
Patent document 2: Japanese Patent Application National Publication No. 2005-511807
Patent document 3: Japanese Patent Application National Publication No. Hei-11-509856

Methods for preparing an aromatic polymer by a coupling reaction of a boronic acid ester, formed from the corresponding diboronic acid and a dihydric alcohol, with a bromine-substituted compound in the presence of an alkali, a palladium catalyst and a phosphine are disclosed in WO 2012/133874 A1 and WO 2012/133875 A1.

Methods for preparing aromatic polymers via coupling reactions with organic boronic acids in the presence of a transition metal catalyst are further disclosed in WO 2009/054832 A1, the paper of SHARMA, N. ET AL., "Pd-Catalyzed Orthogonal Knoevenagel/Perkin Condensation-Decarboxylation-Heck/Suzuki Sequences: Tandem Transformations of Benzaldehydes into Hydroxy-Functionalized Antidiabetic Stilbene-Cinnamoyl Hybrids and Asymmetric Distyrylbenzenes", CHEMISTRY - A EUROPEAN JOURNAL, (2011), vol. 17, no. 37, pages 10350-10356, the paper of NIFANT'EV, I.E. ET AL., "Asymmetric ansa-Zirconocenes Containing a 2-Methyl-4-aryltetra-hydroindacene Fragment: Synthesis, Structure, and Catalytic Activity in Propylene Polymerization and Copolymerization", ORGANOMETALLICS, (2011), vol. 30, no. 21, pages 5744-5752, and in WO 2004/106351 A1.

WO 98/40331 A1 describes a method for preparing indanones via a coupling reaction with aromatic boronic acids and by using a transition metal catalyst.

Coupling reactions of an aromatic boronic acid with a bromo or chloro-substituted aromatic compound in the presence of alkali, ethylene glycpl and a palladium catalyst are disclosed in WO 2005/058916 A2 and US 6 627 764 B1.

In WO 2008/080992 A2 is disclosed the reaction of an aromatic boronic acid with pinacol or ethylene glycol for preparing a cyclic ester.

The reaction of an aromatic boronic acid or boronic acid ester with a halogen substituted aromatic compound in the presence of an aromatic phopspine compound, an aromatic phosphonium salt, a base and a palladium catalyst is disclosed in WO 2014/007404 A1.

Further, MONOPOLI, ANTONIO et al., "Palladium/zirconium oxide nanocomposite as a highly recyclable catalyst for C-C coupling reactions in water", Molecules, (2010), vol. 15, pages 4511-4525, describe a palladium/zirconium oxide nanocomposite as a catlyst for coupling reactions of aromatic boron acids with halogen-substituted aromatic compounds in the presence of tetra(n-butyl)ammonium hydroxide.

### Summary of the Invention

### Problem to be Solved by the Invention

The compound production methods described in the prior art described above, however, have not necessarily sufficient reaction rate.

Then, the present invention has an object of providing a compound production method which is excellent in reaction rate (having higher reaction rate) .

### Means for Solving the Problem

The present invention provides the following [1] to [3] .

[1] A method for producing a compound, comprising:
   a step of supplying
      a polymer compound (1), wherein the polymer compound (1) is a polymer compound represented by the formula (11): 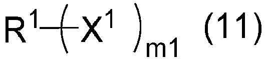 wherein
         m1 represents an integer of 1 to 3,
         R¹ represents a group obtained from a polymer organic compound by removing m1 hydrogen atom(s), and
         X¹ represents an atom or a group selected from Group A, wherein the Group A contains a chlorine atom, a bromine atom, an iodine atom and a group represented by -O-S(=O)₂R^{C1}, wherein R^{C1} represents an alkyl group, a cycloalkyl group or an aryl group, the foregoing groups each optionally having a substituent,
      a compound (2), wherein the compound (2) is a compound represented by the formula (12): 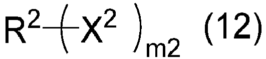 wherein
         m2 represents 1,
         R² represents a group obtained from an aromatic hydrocarbon, an aromatic heterocyclic compound or an aromatic amine by removing m2 hydrogen atom(s) attached directly to carbon atoms constituting the ring thereof, and
         X² represents a group represented by -B(OH)₂,
      a transition metal catalyst,
      an organic base selected from among (CH₃)₄NOH, (C₂H₅) ₄NOH, (C₃H₇) ₄NOH, (C₄H₉) ₄NOH, (C₅H₁₁) ₄NOH, (C₁₆H₃₃) (CH₃) ₃NOH, (C₈H₁₇)₃(CH₃) NOH, (C₈H₁₇)₂(C₁₀H₂₁) (CH₃) NOH, (C₈H₁₇) (C₁₀H₂₁) ₂ (CH₃) NOH, (C₁₀H₂₁) ₃ (CH₃) NOH and (C₈H₁₇) ₄NOH, and
      a compound having two or more hydroxyl groups represented by any one of the formula (4-1) to the formula (4-8) 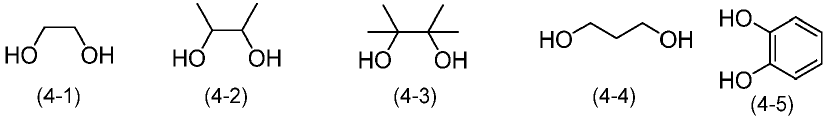 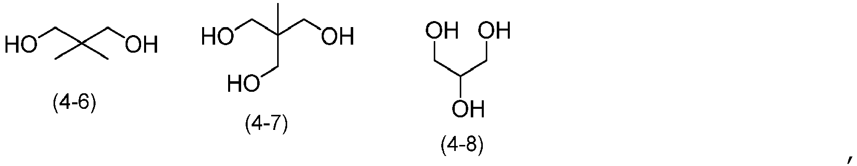 into a reaction vessel, and
   a step of, in the reaction vessel reacting the compound (1) with the compound (2)
   in the presence of the transition metal catalyst, the organic base and the compound having two or more hydroxyl groups,
   wherein the atom or group selected from group (A) in polymer compound (1) and the group represented by ―B(OH)₂ in compound (2) conduct a coupling reaction to obtain a reaction product being a polymer organic compound.
[2] The method for producing a polymer compound according to [1], wherein the at least one atom or group selected from Group A is a chlorine atom, a bromine atom, a mesylate group or a trifluoromethanesulfonate group.
[3] The method for producing a polymer compound according to any one of [1] to [2], wherein the transition metal catalyst is a palladium catalyst.

### Effect of the Invention

According to the present invention, a compound production method excellent in reaction rate (having higher reaction rate) can be produced.

### Modes for Carrying Out the Invention

Suitable embodiments of the present invention will be illustrated in detail below.

### <Explanation of common terms>

Terms commonly used in the present specification have the following meanings unless otherwise stated.

Me represents a methyl group, Et represents an ethyl group, Bu represents a butyl group, i-Pr represents an isopropyl group and t-Bu represents a tert-butyl group.

A hydrogen atom may be a heavy hydrogen atom or a light hydrogen atom.

In the formula representing an organometallic compound, a solid line representing a bond to a central metal denotes a covalent bond or a coordinate bond.

"Polymer organic compound" denotes a polymer having molecular weight distribution and having a polystyrene-equivalent number average molecular weight of 1×10³ to 1×10⁸.

"Constitutional unit" denotes a unit structure found once or more in a polymer organic compound.

"Alkyl group" may be any of linear or branched. The number of carbon atoms of the linear alkyl group is, not including the number of carbon atoms of a substituent, usually 1 to 50, preferably 3 to 30, more preferably 4 to 20. The number of carbon atoms of branched alkyl group is, not including the number of carbon atoms of a substituent, usually 3 to 50, preferably 3 to 30, more preferably 4 to 20.

The alkyl group optionally has a substituent, and examples thereof include a methyl group, an ethyl group, a propyl group, an isopropyl group, a butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group, and groups obtained by substituting a hydrogen atom in these groups with a cycloalkyl group, an alkoxy group, a cycloalkoxy group, an aryl group, a fluorine atom or the like, and the alkyl group having a substituent includes a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group and a 6-ethyloxyhexyl group.

The number of carbon atoms of "Cycloalkyl group" is, not including the number of carbon atoms of a substituent, usually 3 to 50, preferably 3 to 30, more preferably 4 to 20.

The cycloalkyl group optionally has a substituent, and examples thereof include a cyclohexyl group, a cyclohexylmethyl group and a cyclohexylethyl group.

"Aryl group" denotes an atomic group remaining after removing from an aromatic hydrocarbon one hydrogen atom linked directly to a carbon atom constituting the ring. The number of carbon atoms of the aryl group is, not including the number of carbon atoms of a substituent, usually 6 to 60, preferably 6 to 20, more preferably 6 to 10.

The aryl group optionally has a substituent, and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group, a 4-phenylphenyl group, and groups obtained by substituting a hydrogen atom in these groups with an alkyl group, a cycloalkyl group, an alkoxy group, a cycloalkoxy group, an aryl group, a fluorine atom or the like.

"Alkoxy group" may be any of linear or branched. The number of carbon atoms of the linear alkoxy group is, not including the number of carbon atoms of a substituent, usually 1 to 40, preferably 4 to 10. The number of carbon atoms of the branched alkoxy group is, not including the number of carbon atoms of the substituent, usually 3 to 40, preferably 4 to 10.

The alkoxy group optionally has a substituent, and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, an octyloxy group, a 2-ethylhexyloxy group, a nonyloxy group, a decyloxy group, a 3,7-dimethyloctyloxy group and a lauryloxy group, and groups obtained by substituting a hydrogen atom in these groups with a cycloalkyl group, an alkoxy group, a cycloalkoxy group, an aryl group, a fluorine atom or the like.

The number of carbon atoms of "Cycloalkoxy group" is, not including the number of carbon atoms of a substituent, usually 3 to 40, preferably 4 to 10.

The cycloalkoxy group optionally has a substituent, and examples thereof include a cyclohexyloxy group.

The number of carbon atoms of "Aryloxy group" is, not including the number of carbon atoms of a substituent, usually 6 to 60, preferably 7 to 48.

The aryloxy group optionally has a substituent, and examples thereof include a phenoxy group, a 1-naphthyloxy group, a 2-naphthyloxy group, a 1-anthracenyloxy group, a 9-anthracenyloxy group, a 1-pyrenyloxy group, and groups obtained by substituting a hydrogen atom in these groups with an alkyl group, a cycloalkyl group, an alkoxy group, a cycloalkoxy group, a fluorine atom or the like.

The number of carbon atoms of hydrocarbon, not including the number of carbon atoms of the substituent, usually 1 to 60, preferably 6 to 20, more preferably 6 to 14. The hydrocarbon includes, for example, an aliphatic hydrocarbon and an aromatic hydrocarbon.

"Aliphatic hydrocarbon" includes, for example, methane, ethane, propane, butane, isobutane, pentane, 2-methylbutane, 2-ethylbutane, hexane, heptane, octane, 2-ethylhexane, 3-propylheptane, decane, 3,7-dimethyloctane, 2-ethyloctane, 2-hexyldecane and dodecane.

"Aromatic hydrocarbon" includes, for example, benzene, naphthalene, anthracene, phenanthrene, dihydrophenanthrene, naphthacene, fluorene, pyrene, perylene and chrysene.

The number of carbon atoms of heterocyclic compound is, not including the number of carbon atoms of the substituent, usually 2 to 60, preferably 4 to 20, more preferably 4 to 10. The heterocyclic compound includes, for example, an aromatic heterocyclic compound.

"Aromatic heterocyclic compound" includes, for example, compounds in which the heterocyclic ring itself shows aromaticity such as oxadiazole, thiadiazole, thiazole, oxazole, thiophene, pyrrole, phosphole, furan, pyridine, pyrazine, pyrimidine, triazine, pyridazine, quinoline, isoquinoline, carbazole, dibenzophosphole or the like, and compounds in which an aromatic ring is condensed to a heterocyclic ring even if the heterocyclic ring itself shows no aromaticity such as phenoxazine, phenothiazine, dibenzoborole, dibenzosilole, benzopyran or the like.

"q-Valent heterocyclic group*"* (q represents an integer of 1 or more) denotes an atomic group remaining after removing from a heterocyclic compound q hydrogen atoms among hydrogen atoms directly linked to a carbon atom or a hetero atom constituting the ring. Of q-valent heterocyclic groups, "q-valent aromatic heterocyclic groups" as an atomic group remaining after removing from an aromatic heterocyclic compound q hydrogen atoms among hydrogen atoms directly linked to a carbon atom or a hetero atom constituting the ring are preferable.

The number of carbon atoms of the monovalent heterocyclic group is, not including the number of carbon atoms of the substituent usually 2 to 60, preferably 4 to 20.

The monovalent heterocyclic group optionally has a substituent, and examples thereof include a thienyl group, a pyrrolyl group, a furyl group, a pyridyl group, a piperidinyl group, a quinolinyl group, an isoquinolinyl group, a pyrimidinyl group, a triazinyl group, and groups obtained by substituting a hydrogen atom in these groups with an alkyl group, a cycloalkyl group, an alkoxy group, a cycloalkoxy group or the like.

"Halogen atom*"* denotes a fluorine atom, a chlorine atom, a bromine atom or an iodine atom.

"Amino group*"* optionally has a substituent, and a substituted amino group is preferable. The substituent which an amino group has is preferably an alkyl group, a cycloalkyl group, an aryl group or a monovalent heterocyclic group.

The substituted amino group includes, for example, a dialkylamino group, a dicycloalkylamino group and a diarylamino group.

The amino group includes, for example, a dimethylamino group, a diethylamino group, a diphenylamino group, a bis(4-methylphenyl)amino group, a bis(4-tert-butylphenyl)amino group and a bis(3,5-di-tert-butylphenyl)amino group.

"Alkenyl group" may be any of linear or branched. The number of carbon atoms of the linear alkenyl group, not including the number of carbon atoms of the substituent, usually 2 to 30, preferably 3 to 20. The a number of carbon atoms of branched alkenyl group is, not including the number of carbon atoms of the substituent, usually 3 to 30, preferably 4 to 20.

The number of carbon atoms of "Cycloalkenyl group", not including the number of carbon atoms of the substituent, is usually 3 to 30, preferably 4 to 20.

The alkenyl group and the cycloalkenyl group each optionally have a substituent, and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group, a 7-octenyl group, and these groups having a substituent.

"Alkynyl group*"* may be any of linear or branched. The number of carbon atoms of the alkynyl group, not including carbon atoms of the substituent, is usually 2 to 20, preferably 3 to 20t. The number of carbon atoms of the branched alkynyl group, not including carbon atoms of the substituent, is usually 4 to 30, preferably 4 to 20.

The number of carbon atoms of "Cycloalkynyl group", not including carbon atoms of the substituent, is usually 4 to 30, preferably 4 to 20.

The alkynyl group and the cycloalkynyl group each optionally have a substituent, and examples thereof include an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and these groups having a substituent.

"Crosslinkable group*"* is a group capable of forming a new bond by being subjected to a heating treatment, an ultraviolet irradiation treatment, a radical reaction or the like, and is preferably a group represented by any one of the formulae (B-1) to (B-17). The foregoing groups each optionally have a substituent.

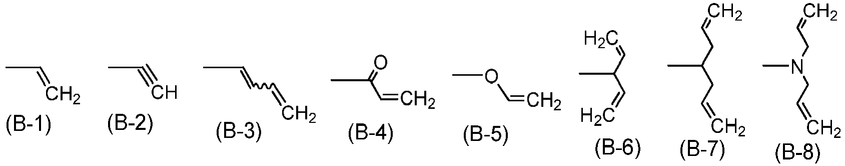

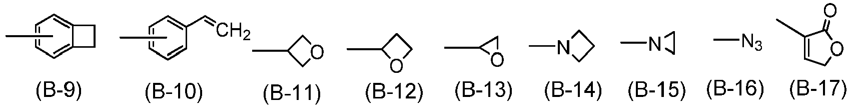

"Substituent" represents a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group or a cycloalkynyl group. The substituent may be a crosslinkable group.

"Constitutional unit represented by the formula (X)" is a constitutional unit which a polymer organic compound optionally contains. When a polymer organic compound contains a constitutional unit represented by the formula (X), the constitutional unit represented by the formula (X) may be contained each singly or two or more constitutional units may be contained. 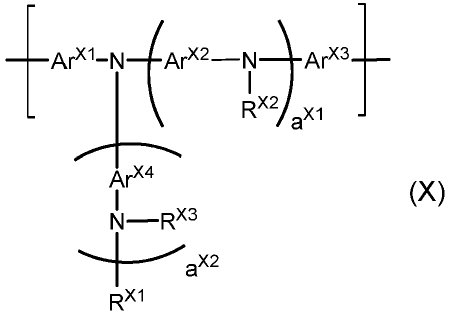 [wherein,
a^{X1} and a^{X2} each independently represent an integer of 0 or more.
Ar^{X1} and Ar^{X3} each independently represent an arylene group or a divalent heterocyclic group, the foregoing groups each optionally having a substituent.
Ar^{X2} and Ar^{X4} each independently represent an arylene group, a divalent heterocyclic group or a divalent group in which at least one arylene group and at least one divalent heterocyclic group are bonded directly, the foregoing groups each optionally having a substituent. When a plurality of Ar^{X2} and Ar^{X4} are present, they may be the same or different at each occurrence.
R^{X1}, R^{X2} and R^{X3} each independently represent a hydrogen atom, an alkyl group, an aryl group or a monovalent heterocyclic group, the foregoing groups each optionally having a substituent. When a plurality of R^{X2} and R^{X3} are present, they may be the same or different at each occurrence.].

The constitutional unit represented by the formula (X) includes, for example, constitutional units represented by the formulae (X1-1)-(X1-11).

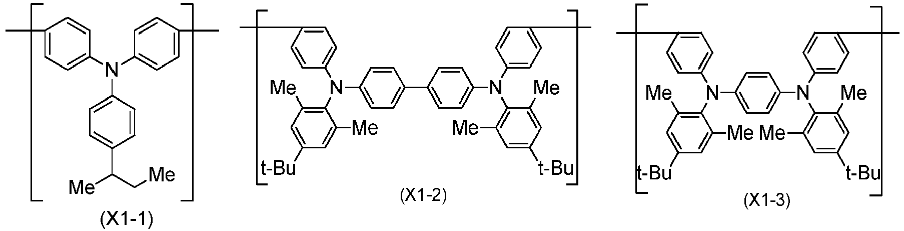

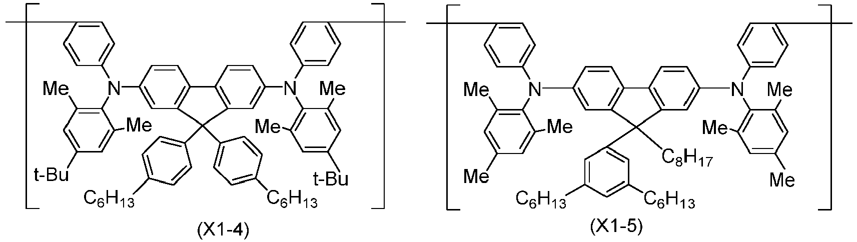

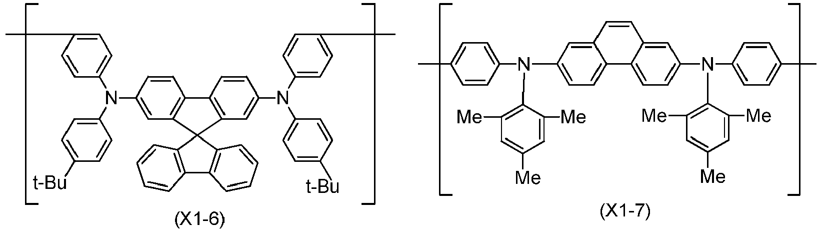

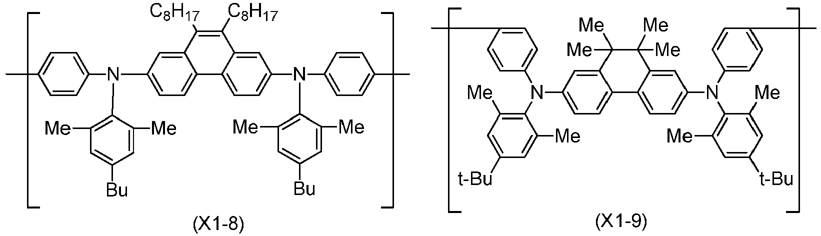

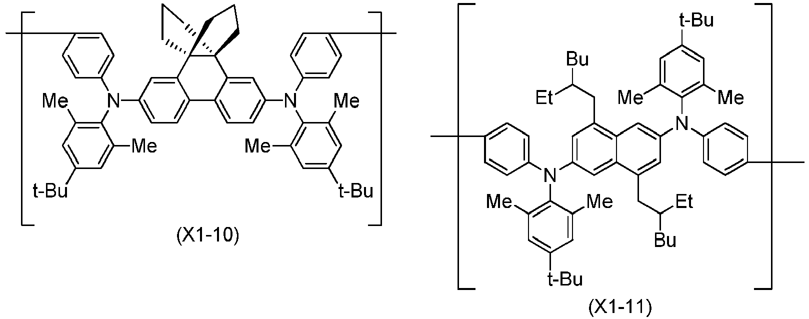

"Constitutional unit represented by the formula (Y)" is a constitutional unit which a polymer organic compound may contain. When a polymer organic compound contains a constitutional unit represented by the formula (Y), the constitutional unit represented by the formula (Y) may be contained each singly or two or more constitutional units may be contained. 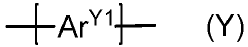 [wherein, Ar^{Y1} represents an arylene group, a divalent heterocyclic group or a divalent group in which at least one arylene group and at least one divalent heterocyclic group are bonded directly, the foregoing groups each optionally having a substituent.].

The constitutional unit represented by the formula (Y) includes, for example, constitutional units composed of arylene groups represented by the formulae (Y-101)-(Y-121), constitutional units composed of divalent heterocyclic groups represented by the formulae (Y-201)-(Y-206), and constitutional units composed of divalent groups in which at least one arylene group and at least one divalent heterocyclic group are bonded directly represented by the formulae (Y-301)-(Y-304).

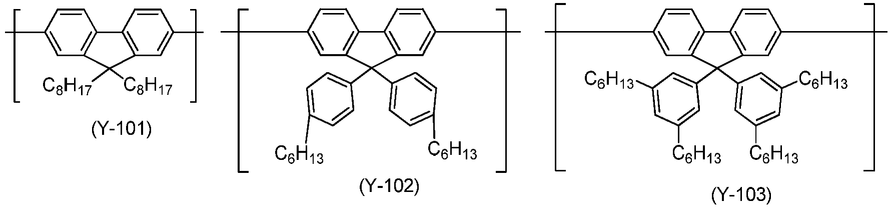

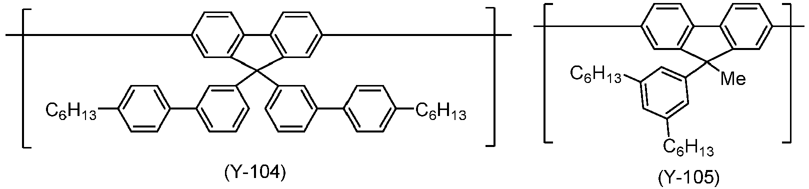

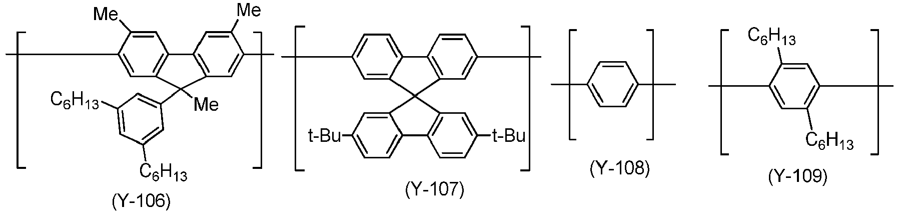

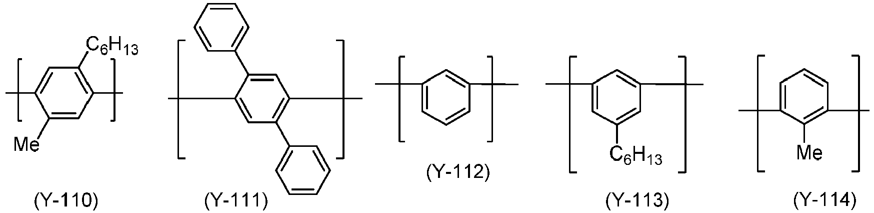

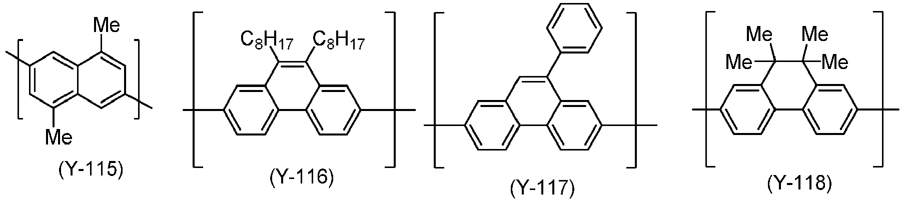

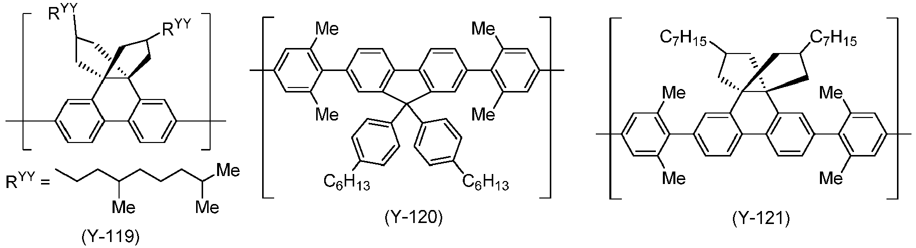

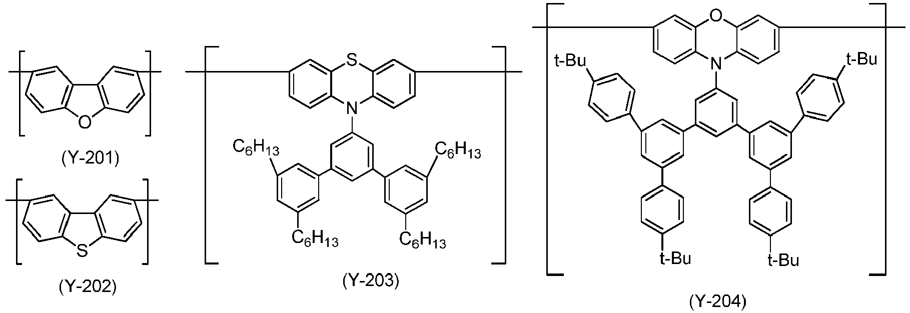

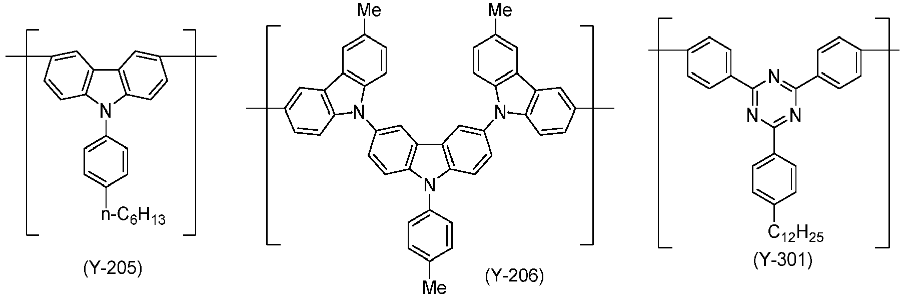

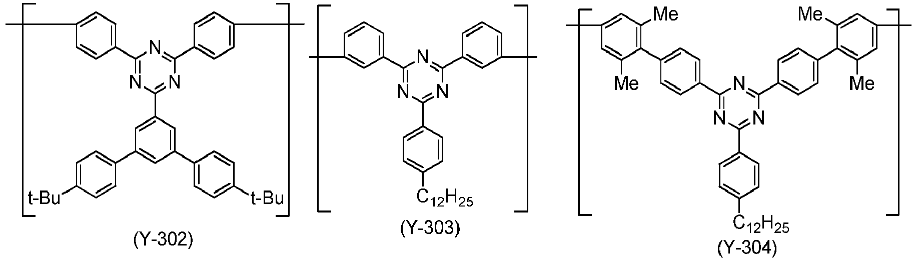

"Group obtained from an organometallic compound by removing r hydrogen atoms" (r represents an integer of 1 or more.) denotes an atomic group remaining after removing from an organometallic compound r hydrogen atoms among hydrogen atoms bonding to carbon atoms or hetero atoms constituting the organometallic compound.

The organometallic compound includes, for example, phosphorescent compounds, and preferable are phosphorescent compounds represented by the formula (Z) . 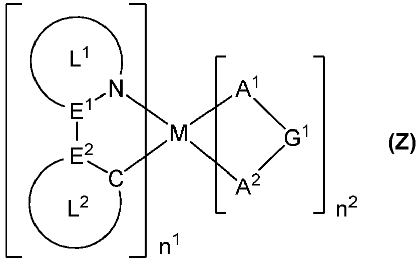 [wherein,
M represents an iridium atom or a platinum atom.
n¹ represents an integer of 1 or more, and n² represents an integer of 0 or more. n¹+n² is 3 when M is an iridium atom and n¹+n² is 2 when M is a platinum atom.
E¹ and E² each independently represent a carbon atom or a nitrogen atom. At least one of E¹ and E² is a carbon atom.
A ring L¹ represents an aromatic heterocyclic ring, the foregoing ring each optionally having a substituent. When a plurality of the substituents are present, they may be the same or different, and may be combined together to form a ring together with atoms to which they are attached. When a plurality of the rings L¹ are present, they may be the same or different. When the ring L¹ is a 6-membered aromatic heterocyclic ring, E¹ is a carbon atom.
A ring L² represents an aromatic hydrocarbon ring or an aromatic heterocyclic ring, the foregoing rings each optionally having a substituent. When a plurality of the substituents are present, they may be the same or different, and may be combined together to form a ring together with atoms to which they are attached. When a plurality of the rings L² are present, they may be the same or different. When the ring L² is a 6-membered aromatic heterocyclic ring, E² is a carbon atom.
A¹-G¹-A² represents an anionic bidentate ligand, and A¹ and A² each independently represent a carbon atom, an oxygen atom or a nitrogen atom, and these atoms may be an atom constituting the ring. G¹ represents a single bond or an atomic group constituting a bidentate ligand together with A¹ and A². When a plurality of A¹-G¹-A² are present, they may be the same or different.].

The phosphorescent compound represented by the formula (Z) includes, for example, phosphorescent compounds represented by the following formulae.

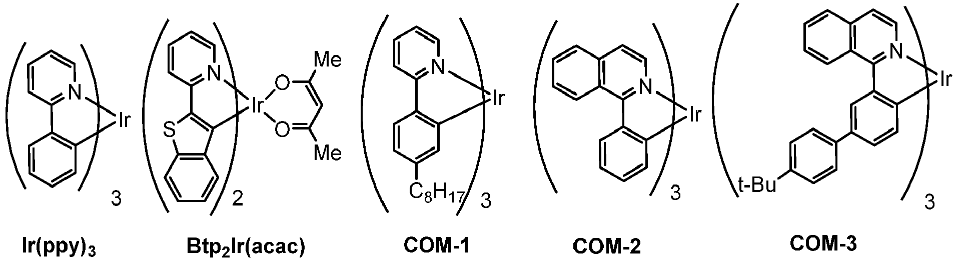

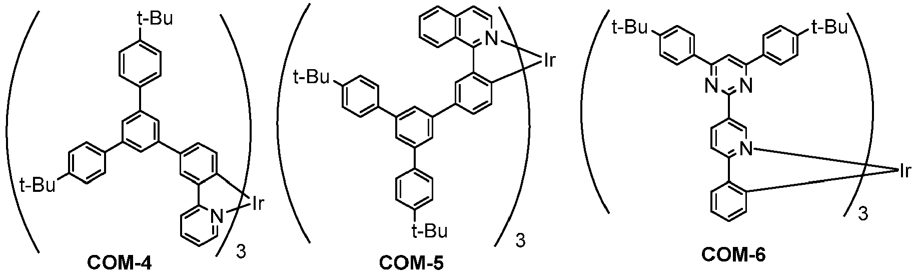

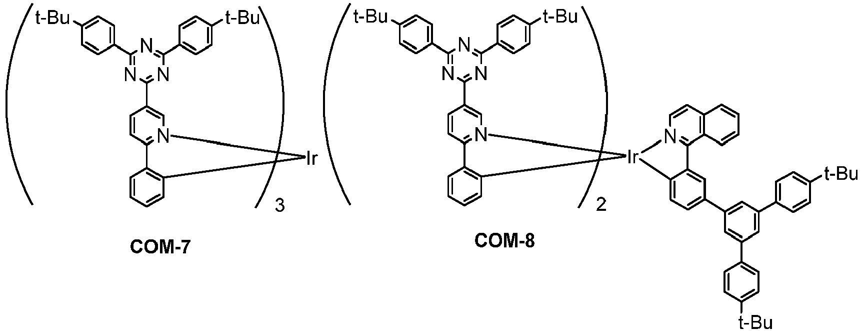

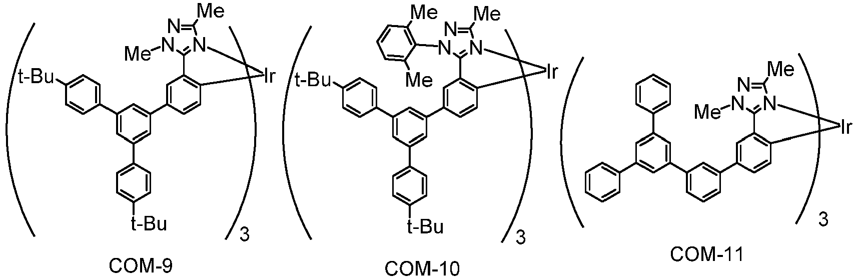

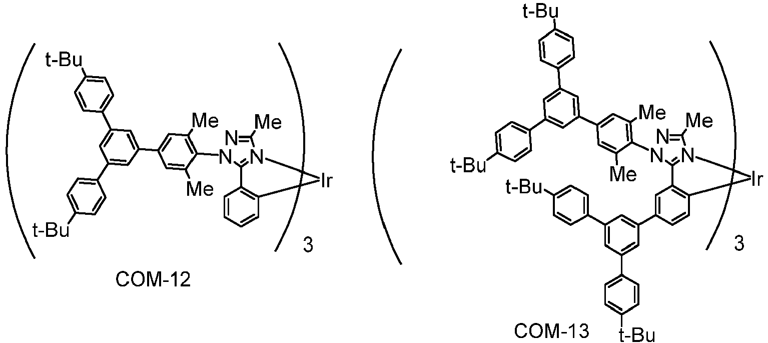

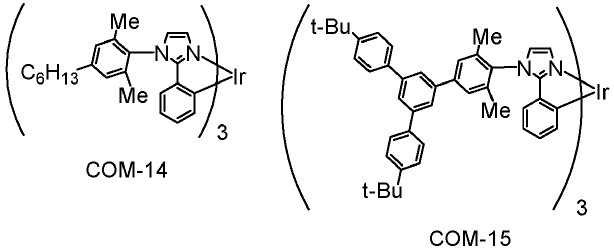

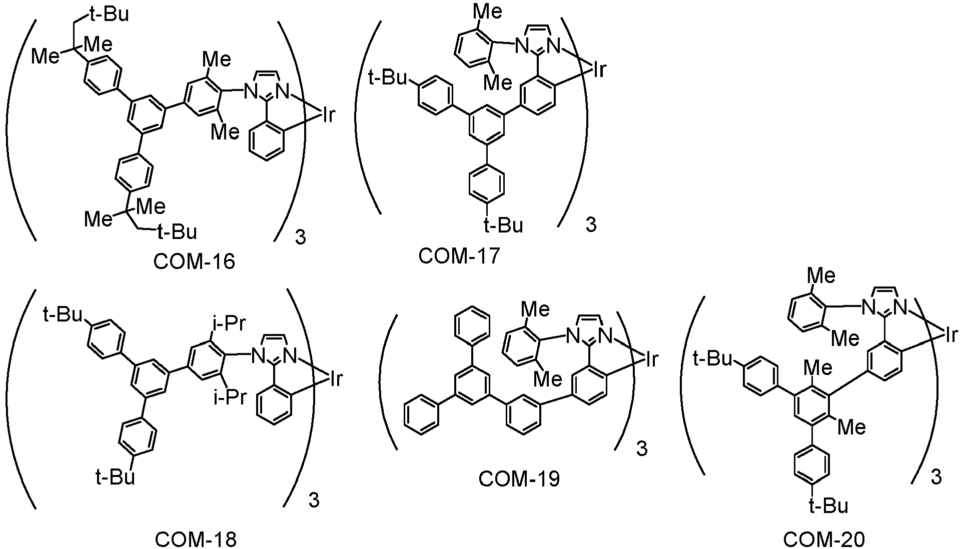

The present invention is a production method of a compound, as defined in claim 1.

### <Polymer Compound (1)>

The polymer compound (1) is a polymer compound having at least one atom or group selected from Group A. The polymer compound (1) is usually a compound which is dissolvable in an organic solvent.

R^{C1} in the group represented by -O-S(=O)₂R^{C1} in Group A is preferably an alkyl group having a number of carbon atoms of 1 to 20 or an aryl group having a number of carbon atoms of 6 to 20.

The group represented by -O-S(=O)₂R^{C1} in Group A includes, for example, a mesylate group, a trifluoromethanesulfonate group and a p-toluenesulfonate group, and preferable is a mesylate group or a trifluoromethanesulfonate group.

The polymer compound (1) is preferably a polymer compound having at least one atom or group selected from the group consisting of a chlorine atom, a bromine atom, a mesylate group and a trifluoromethanesulfonate group, more preferably a compound having at least one atom selected from the group consisting of a chlorine atom and a bromine atom, further preferably a compound having a bromine atom, because the method for producing the present invention is more excellent in the reaction rate.

The polymer compound (1) is a polymer compound represented by the formula (11), which is of easier availability. 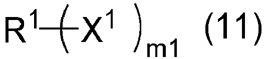 (wherein,
m1 represents an integer of 1 to 3.
R¹ represents a group obtained from a polymer organic compound by removing m1 hydrogen atom(s).
X¹ represents an atom or a group selected from Group A described above.)
m1 is preferably 1 or 2.
X¹ is preferably a chlorine atom, a bromine atom, a mesylate group or a trifluoromethanesulfonate group, more preferably a chlorine atom or a bromine atom, further preferably a bromine atom.
R¹ is a group obtained from a polymer organic compound by removing m1 hydrogen atom(s).
R¹ is a group obtained from a polymer organic compound by removing m1 hydrogen atom(s), wherein examples thereof include groups obtained from a polymer organic compound by removing m1 hydrogen atom(s) containing at least one constitutional unit selected from the group consisting of a constitutional unit represented by the formula (X), a constitutional unit represented by the formula (Y) and a constitutional unit obtained from an organometallic compound by removing two hydrogen atoms, and preferable are groups obtained from a polymer organic compound containing at least one constitutional unit selected from the group consisting of a constitutional unit represented by the formula (X) and a constitutional unit represented by the formula (Y) by removing m1 hydrogen atom(s).

### [Compound (1) (m1 is 1)]

The compound (1) (m1 is 1) includes, for example, polymer organic compounds represented by the following formulae. In the polymer organic compound represented by the following formula, n represents the degree of polymerization, and p, q, (100-p) and (100-p-q) represent molar % of respective constitutional units. The polymer organic compound represented by the following formula may be any of a random copolymer, an alternate copolymer and a block copolymer.

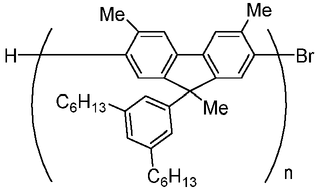

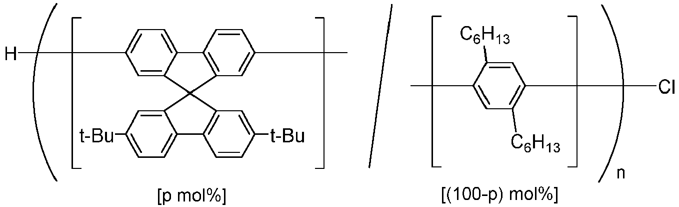

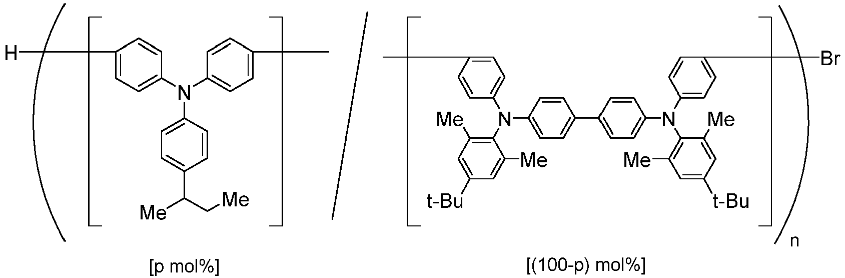

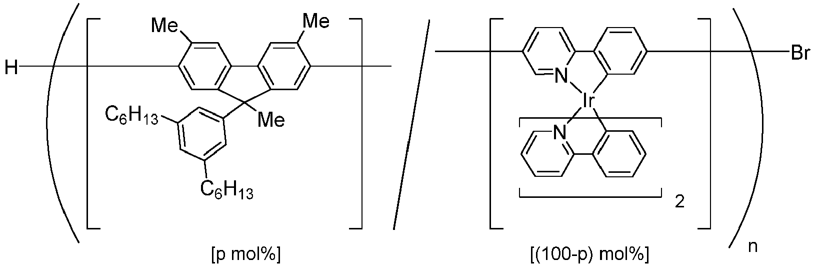

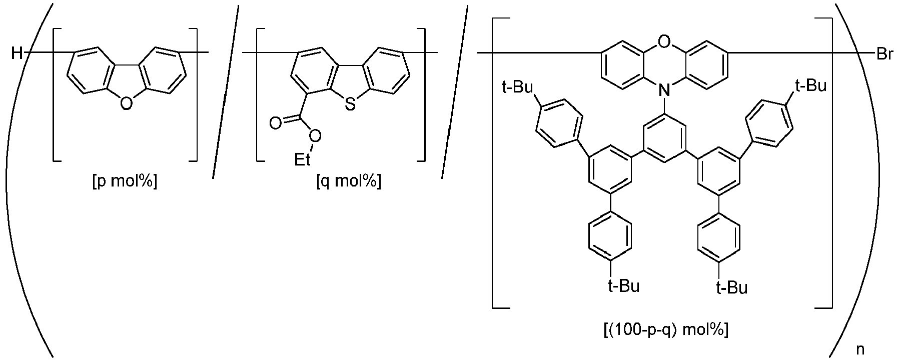

### [Compound (1) (m1 is 2)]

The compound (1) (m1 is 2) includes, for example, polymer organic compounds represented by the following formulae. In the polymer organic compound represented by the following formula, n represents the degree of polymerization, and p, q, (100-p) and (100-p-q) represent molar % of respective constitutional units. The polymer organic compound represented by the following formula may be any of a random copolymer, an alternate copolymer and a block copolymer.

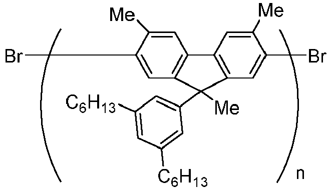

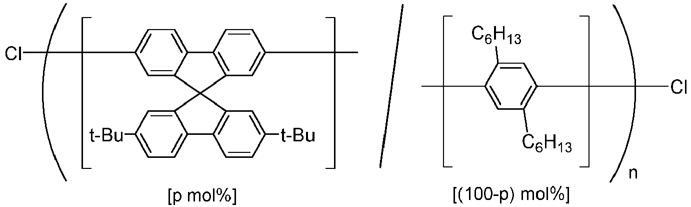

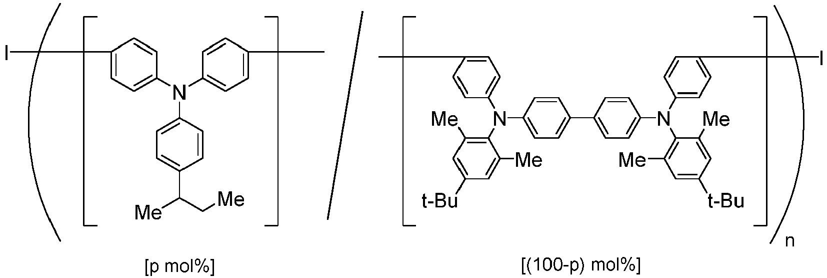

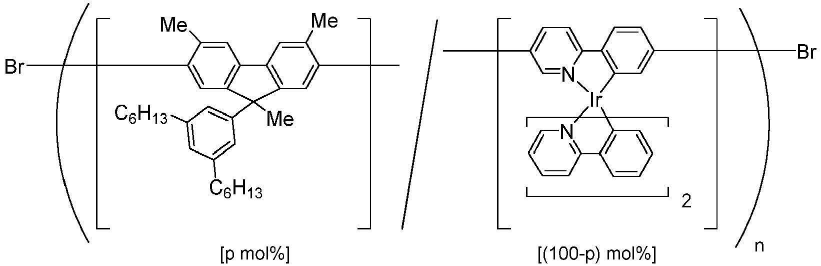

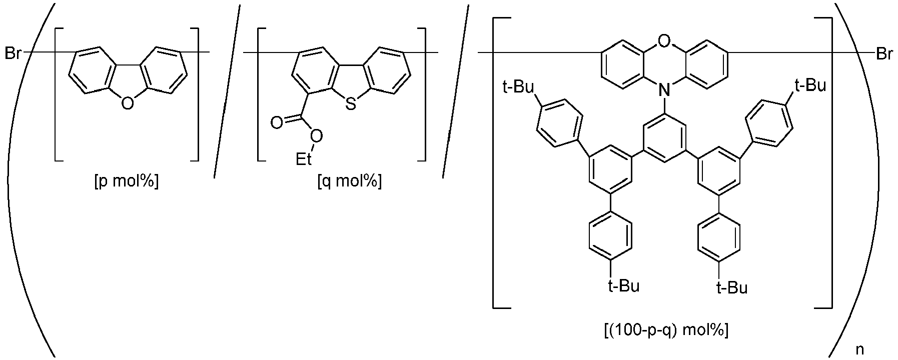

Commercialized products of the compound (1) may be purchased or the compound (1) may be synthesized according to a known method.

### <Compound (2)>

The compound (2) is a compound represented by the formula (12), which is of easier availability. 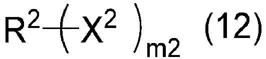 (wherein,
m2 represents 1.
R² represents a group obtained from an aromatic hydrocarbon, an aromatic heterocyclic compound or an aromatic amine by removing m2 hydrogen atom(s) attached directly to carbon atoms constituting the ring thereof.
X² represents a group represented by -B(OH)₂.).
² is a group obtained from an aromatic hydrocarbon, an aromatic heterocyclic compound or an aromatic amine by removing m2 hydrogen atom(s) attached directly to carbon atoms constituting the ring thereof, because the method for producing the present invention is more excellent in the reaction rate. The aromatic hydrocarbon, the aromatic heterocyclic compound and the aromatic amine optionally have a substituent.
When R² is a group obtained from an aromatic hydrocarbon by removing m2 hydrogen atom(s), the hydrocarbon has a number of carbon atoms of usually up to 60, preferably 6 to 20, more preferably 6 to 14, not including the number of carbon atoms of the substituent.
When R² is a group obtained from an aromatic heterocyclic compound by removing m2 hydrogen atom(s), the heterocyclic compound has a number of carbon atoms of usually 2 to 60, preferably 4 to 20, more preferably 4 to 10, not including the number of carbon atoms of the substituent.
When R² is a group obtained from an aromatic amine by removing m2 hydrogen atom(s), the amine has a number of carbon atoms of usually up to 60, preferably 6 to 30, more preferably 6 to 18, not including the number of carbon atoms of the substituent.

### [Compound (2) (m2 is 1)]

The compound (2) (m2 is 1) includes, for example, compounds represented by the following formulae.

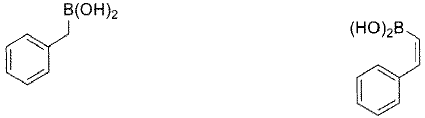

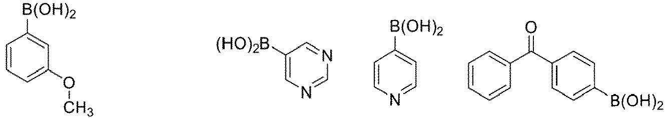

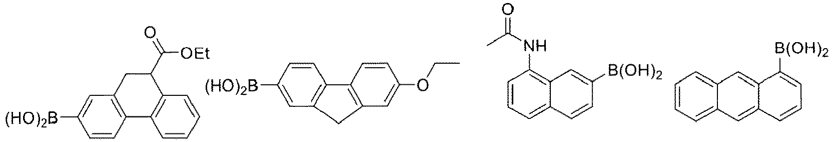

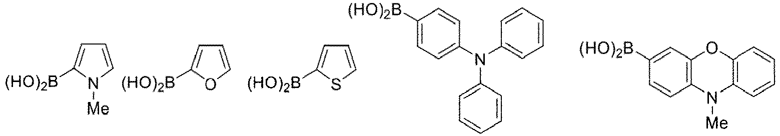

The compound (2) (m2 is 1) includes, for example, also compounds represented by the following formulae.

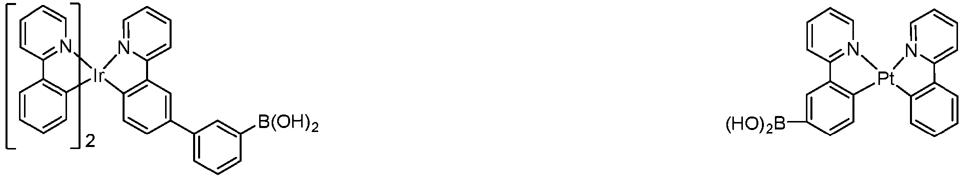

Commercialized products of the compound (2) may be purchased or the compound (2) may be synthesized according to a known method.

### <Transition metal catalyst>

The transition metal catalyst is preferably a Group 10 transition metal catalyst. The Group 10 transition metal catalyst includes, for example, a nickel catalyst, a palladium catalyst and a platinum catalyst, and preferable is a palladium catalyst because the methods for producing the present invention are more excellent in the reaction rate.

The palladium catalyst includes, for example, palladium complexes such as dichlorobis(triphenylphosphine)palladium, dichlorobis[tris(2-methoxyphenyl)phosphine]palladium, palladium[tetrakis(triphenylphosphine)], [tris(dibenzylideneacetone)]dipalladium, dichlorobis[dicyclopentyl(2-methoxyphenyl)phosphine]palladium, palladium acetate, dichlorobis[dicyclopentyl(2, 6-dimethoxyphenyl)phosphine]palladium, bis[di-tert-butyl(3, 5-di-tert-butylphenyl)phosphine]dichloropalladium or the like, and dichlorobis(triphenylphosphine)palladium or palladium[tetrakis(triphenylphosphine)] is preferable. These palladium complexes may be a complex further having a ligand such as triphenylphosphine, tri(tert-butylphosphine), tricyclohexylphosphine, diphenylphosphinopropane, bipyridyl or the like.

In the methods for producing the present invention, the transition metal catalyst may be used each singly or two or more transition metal catalysts may be used in combination.

In the method for producing the present invention, the use amount of the transition metal catalyst is usually 0.00001 to 3 mol with respect to 1 mol of the total use amount of the compound (1) and the compound (2) .

### <Organic base>

The organic base is selected from among (CH₃)₄NOH (tetramethylammonium hydroxide), (C₂H₅)₄NOH (tetraethylammonium hydroxide), (C₃H₇)₄NOH (tetrapropylammonium hydroxide), (C₄H₉)₄NOH (tetrabutylammonium hydroxide), (C₅H₁₁)₄NOH (tetrabutylammonium hydroxide), (C₁₆H₃₃) (CH₃)₃NOH (hexadecyltrimethylammonium hydroxide), (C₈H₁₇)₃(CH₃)NOH (trioctylmethylammonium hydroxide), (C₈H₁₇)₂(C₁₀H₂₁) (CH₃)NOH (dioctyldecylmethylammonium hydroxide), (C₁₀H₂₁)₃(CH₃)NOH (tridecylmethylammonium hydroxide) and (C₈H₁₇)₄NOH (tetraoctylammonium hydroxide) .

In the methods for producing the present invention, the organic base may be used each singly or two or more organic bases may be used in combination.

In the method for producing the present invention, the use amount of the organic base is usually 0.5 to 100 mol, preferably 0.5 to 70 mol, more preferably 0.5 to 40 mol, further preferably 0.9 to 20 mol with respect to 1 mol of the total use amount of the compound (1) and the compound (2).

### <Compound having two or more hydroxyl groups>

The compound having two or more hydroxyl groups is usually a compound which is dissolvable in an organic solvent or water.

The compound having two or more hydroxyl groups is represented by any one of the formula (4-1) to the formula (4-8)

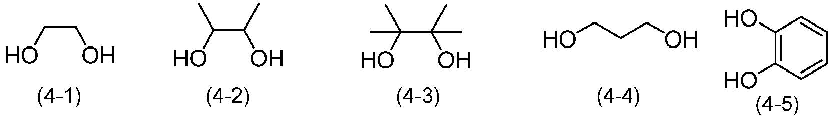

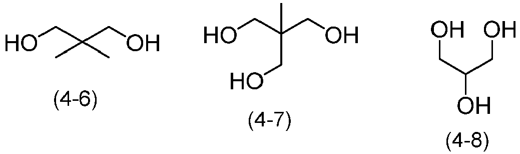

The compound having two or more hydroxyl groups includes a compound represented by any one of the formula (4-1) to the formula (4-8), because the methods for producing the present invention are more excellent in the reaction rate.

In the methods for producing the present invention, the compound having two or more hydroxyl groups may be used each singly or two or more compounds may be used in combination.

In the method for producing the present invention, the use amount of the compound having two or more hydroxyl groups is usually 0.01 to 100 mol, preferably 0.01 to 10 mol, more preferably 0.1 to 1 mol with respect to 1 mol of the total use amount of the compound (1) and the compound (2).

### <Organic solvent>

The methods for producing the present invention are preferably conducted in the presence of an organic solvent.

The method for producing the present invention is a production method of a polymer compound, comprising
a step of supplying
the compound (1),
the compound (2),
the transition metal catalyst, the organic base, the compound having two or more hydroxyl groups and an organic solvent into a reaction vessel, and
a step of, in the reaction vessel, reacting the compound (1) with the compound (2) in the presence of the transition metal catalyst, the organic base, the compound having two or more hydroxyl groups and the organic solvent.

The organic solvent includes, for example, aromatic hydrocarbon solvents such as toluene, xylene, mesitylene or the like; ether solvents such as tetrahydrofuran, 1,4-dioxane, dimethoxyethane or the like; amide solvents such as N,N-dimethylacetamide, N,N-dimethylformamide or the like; alcohol solvents such as methanol, ethanol, isopropyl alcohol, methylcellosolve, butylcellosolve or the like; ketone solvents such as acetone, methyl ethyl ketone, cyclopentanone, cyclohexanone, methyl amyl ketone, methyl isobutyl ketone, N-methyl-2-pyrrolidinone or the like; aliphatic hydrocarbon solvents such as pentane, hexane, heptane or the like; nitrile solvents such as acetonitrile or the like; halogenated hydrocarbon solvents such as chloroform or the like.

The organic solvent is preferably one which is not mixed with water at any ratio. Specifically, aromatic hydrocarbon solvents or ether solvents are preferable, and toluene, xylene or mesitylene is more preferable.

In the methods for producing the present invention, the organic solvent may be used each singly or two or more organic solvents may be used in combination.

In the method for producing the present invention, the use amount of the organic solvent is usually 10 parts by mass to 100000 parts by mass, preferably 1000 parts by mass to 50000 parts by mass, more preferably 2000 parts by mass to 20000 parts by mass with respect to 100 parts by mass of the total use amount of the compound (1) and the compound (2).

### <Reaction vessel>

In the methods for producing the present invention, the reaction vessel is not particularly restricted, and examples thereof include a reaction vessel made of glass, a reaction vessel made of stainless steel and a reaction vessel made of a fluorine resin. The shape of the reaction vessel is not particularly restricted, and examples thereof include a spherical reaction vessel and a cylindrical reaction vessel.

### <Supplying step>

In the method for producing the present invention,
the temperature in the supplying step may be the same as or different from the temperature in the step of reacting the compound (1) with the compound (2) described later.

In the method for producing the present invention,
the pressure in the supplying step is usually atmospheric pressure.

In the method for producing the present invention,
the supplying order in the supplying step is not particularly restricted. That is, the compound (1), the compound (2), a transition metal catalyst, an organic base and a compound having two or more hydroxyl groups may be supplied in a mass into a reaction vessel, alternatively, some materials selected from the group consisting of the compound (1), the compound (2), a transition metal catalyst, an organic base and a compound having two or more hydroxyl groups may be supplied into a reaction vessel, then, the remaining materials may be added and mixed while stirring.

### <Reacting step>

In the method for producing the present invention,
the temperature in the step of reacting the compound (1) with the compound (2) in the presence of a transition metal catalyst, an organic base and a compound having two or more hydroxyl groups is usually 0 to 200°C, preferably 20 to 150°C, more preferably 50 to 100°C.

In the method for producing the present invention,
the time of the step of reacting is usually 0.1 to 48 hours, preferably 0.5 to 24 hours, more preferably 0.5 to 8 hours.

In the method for producing the present invention,
the pressure in the step of reacting is usually atmospheric pressure.

### <Use amount or the like>

In the method for producing the present invention,
the use amount of the compound (1) is usually 0.1 to 10 mol, preferably 0.2 to 5 mol, more preferably 0.5 to 2 mol with respect to 1 mol of the use amount the compound (2).

In the method for producing the present invention,
the compound (1) may be used each singly or two or more compounds (1) may be used in combination. The compound (2) may be used each singly or two or more compounds (2) may be used in combination.

### <Reaction product>

In the method for producing the present invention,
at least one atom or group selected from Group A which the compound (1) has and the group represented by ―B(OH)₂ conduct a coupling reaction. As a result, a reaction product in which an atom to which at least one atom or group selected from Group A is bonded in the compound (1) and an atom to which the group represented by ―B(OH)₂ is bonded in the compound (2) are bonded is obtained. The reaction product is a polymer organic compound.

In the method for producing the present invention,
a compound (m1 is 1) represented by the formula (11) and a compound (m2 is 1) represented by the formula (12) are reacted, to obtain a reaction product represented by the formula (21).

R¹-R² (21)

In the method for producing the present invention,
a compound (m1 is 2) represented by the formula (11) and a compound (m2 is 1) represented by the formula (12) are reacted, to obtain a reaction product represented by the formula (22) and a reaction product represented by the formula (23).

X¹-R¹-R² (22)

R²-R¹-R² (23)

The reaction product represented by (21) includes, for example, polymer organic compounds represented by the following formulae. In the polymer organic compounds represented by the following formulae, n represents the degree of polymerization, and p and (100-p) represent molar % of respective constitutional units. The polymer organic compound represented by the following formula may be any of a random copolymer, an alternate copolymer and a block copolymer.

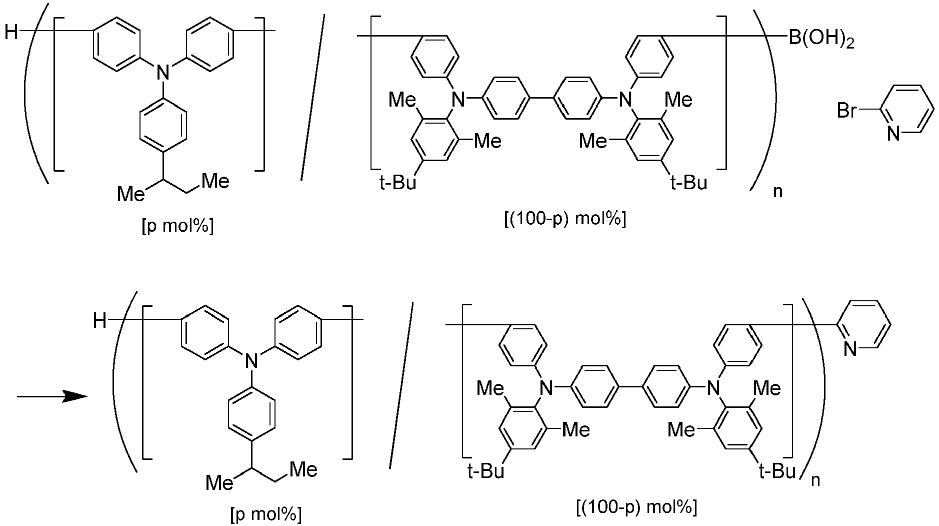

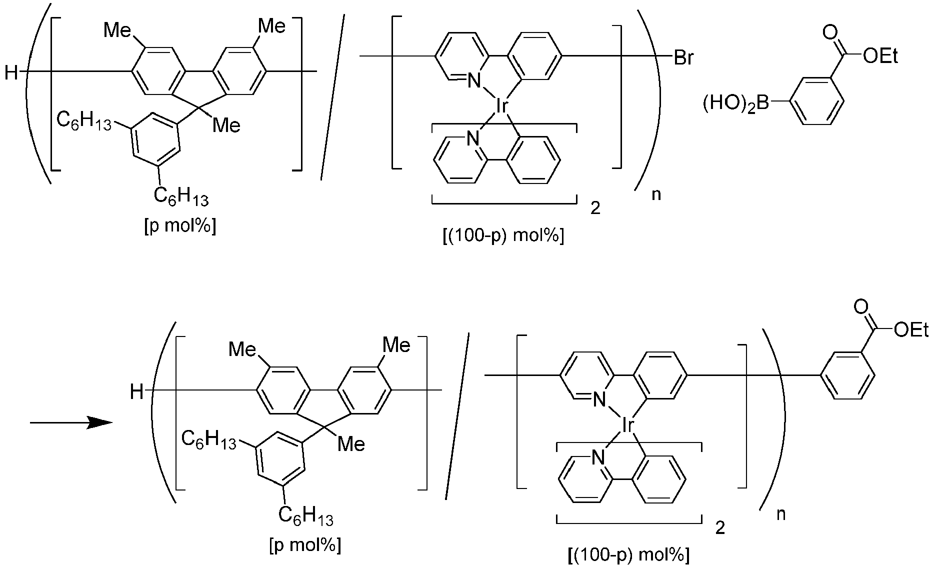

The reaction product represented by (23) includes, for example, polymer organic compounds represented by the following formulae. In the polymer organic compounds represented by the following formulae, n represents the degree of polymerization, and p and (100-p) represent molar % of respective constitutional units. The polymer organic compound represented by the following formula may be any of a random copolymer, an alternate copolymer and a block copolymer.

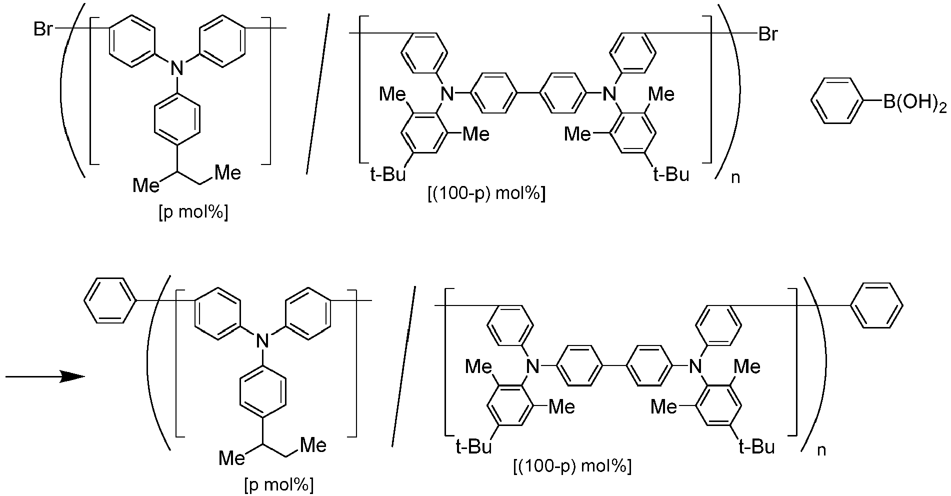

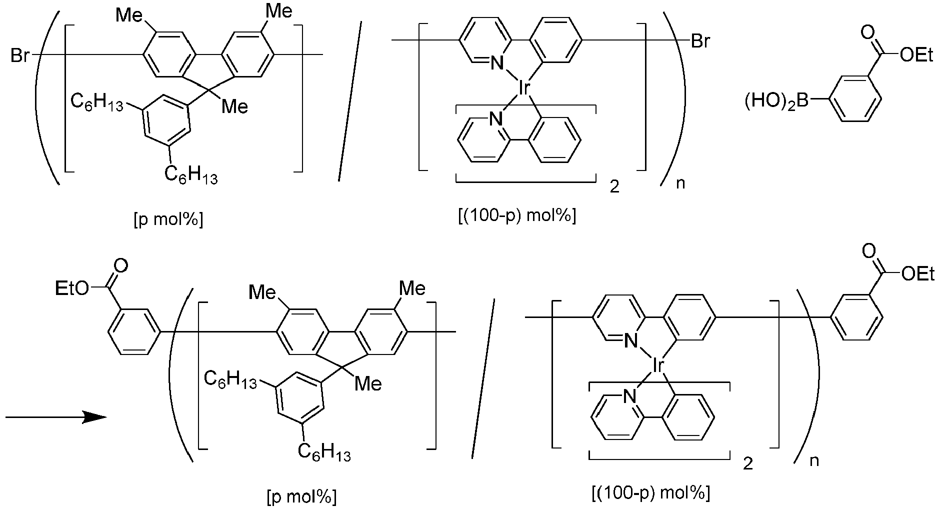

### <Others>

After reaction of the compound (1) with the compound (2), the desired compound can be taken out by known methods such as a method for mixing a reaction mixture and water, separating the mixture, then, concentrating the liquid to dryness, a method for mixing a reaction mixture and a lower alcohol (methanol or the like), isolating the deposited precipitate by filtration, then, drying the precipitate, or the like, in the methods for producing the present invention.

The compound taken out can be purified by known methods such as, for example, recrystallization, reprecipitation, continuous extraction with a Soxhlet extractor, column chromatography, sublimation purification or the like.

### EXAMPLES

The present invention will be illustrated further in detail by examples below.

### <Liquid chromatography (LC) analysis condition>

Measurement Apparatus: CBM-20A (manufactured by Shimadzu Corp.)
Column: L-Column2 ODS (3 µm, 4.6 mmϕ × 25 cm)
Mobile phase A: acetonitrile
Mobile phase B: THF
Mobile phase C: pure water
Gradient: 0.01 minute A = 60%, B = 0%, C = 40%
   10 minutes A = 60%, B = 0%, C = 40%
   20 minutes A = 100%, B = 0%, C = 0%
   100 minutes A = 100%, B = 0%, C = 0%
   120 minutes A = 0%, B = 100%, C = 0%
   130 minutes A = 0%, B = 100%, C = 0%
Flow rate: 1.0 mL/min
Detection wavelength: 280 nm

### <Gel permeation chromatography (GPC) analysis condition>

Measurement Apparatus: HLC-822GPC (manufactured by Tosoh Corporation)
Column: PLgel 10µm MIXED-B (manufactured by Tosoh Corporation)
Column temperature: 40°C
Mobile phase: tetrahydrofuran
Flow rate: 1.5 mL/min
Detection wavelength: 228 nm

### <Gas chromatography (GC) analysis condition>

GC Measurement Apparatus: GC-2010 (manufactured by Shimadzu Corp.)
Column: J&W DB-1 (0.25 mmϕ × 30 m, 1.0 µm)
Column temperature: 40°C (10 minutes) → (10°C/min) → 300°C (20 minutes)
Analysis time (total): 56 minutes
Injection port temperature: 250°C, detector temperature: 250°C (FID)
Carrier gas: helium gas (flow speed: about 2.6 mL/min)
Injection port pressure: 85.3 kPa (control mode: pressure)
Split ratio: total flow rate 43.1 mL/min (split ratio: about 1/15)
Injection amount: 1 µL

Oxygen combustion-ion chromatograph analysis was performed using ICS-2100 (manufactured by Nippon Dionex K.K.).

### <Comparative Example 1> Synthesis of compound (5-1)

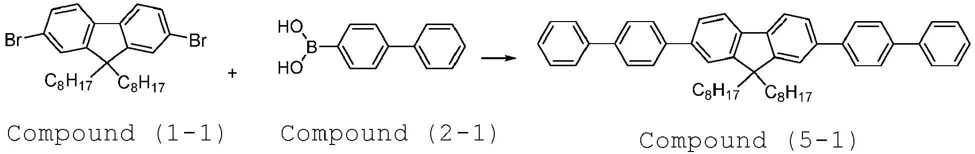

The compound (1-1) was synthesized according to a method described in International Publication WO2002/045184.

As the compound (2-1), 4-biphenylboronic acid manufactured by Tokyo Chemical Industry Co., Ltd. was used.

A nitrogen gas atmosphere was prepared in a reaction vessel, then, the compound (1-1) (1.93 g), the compound (2-1) (2.43 g), phenanthrene (0.316 g) (internal standard substance), dichlorobis[tris(2-methoxyphenyl)phosphine]palladium (1.19 mg) and toluene (100 g) were added, and the mixture was heated at 86°C. Thereafter, to this was added a 20% tetraethylammonium hydroxide aqueous solution (15 g), and the mixture was stirred at 86°C for 2 hours. The resultant reaction mixture was analyzed by LC, to find that the yield based on the internal standard of the compound (5-1) as the target was 71%. The result is shown in Table 1.

The above-described reaction mixture was stirred at 86°C for further 1 hour. The resultant reaction mixture was analyzed by LC, to find that the yield based on the internal standard of the compound (5-1) as the target was 99%.

<Reference Example 1> Synthesis of compound (5-1) The Reference Example 1 is not comprised by the present invention.

The compound (1-1) was synthesized according to a method described in International Publication WO2002/045184.

As the compound (2-1), 4-biphenylboronic acid manufactured by Tokyo Chemical Industry Co., Ltd. was used.

A nitrogen gas atmosphere was prepared in a reaction vessel, then, the compound (1-1) (1.93 g), the compound (2-1) (2.43 g), phenanthrene (0.316 g) (internal standard substance), dichlorobis[tris(2-methoxyphenyl)phosphine]palladium (1.18 mg), toluene (100 g) and pinacol (0.26 g) were added, and the mixture was heated at 86°C. Thereafter, to this was added a 20% tetraethylammonium hydroxide aqueous solution (15 g), and the mixture was stirred at 86°C for 2 hours. The resultant reaction mixture was analyzed by LC, to find that the yield based on the internal standard of the compound (5-1) as the target was 99%. The result is shown in Table 1.

**[Table 1]**

| | compound having two or more hydroxyl groups | reaction time | yield of compound (5-1) |
|---|---|---|---|
| Comparative Example 1 | none | 2 hours | 71% |
| Reference Example 1 | pinacol | 2 hours | 99% |

A comparison of the yield of the compound (5-1) in Comparative Example 1 with the yield of the compound (5-1) in Reference Example 1 denoted that the method for producing the present invention is excellent in the reaction rate.

### <Synthesis Example 1> Synthesis of polymer organic

### compound P-1

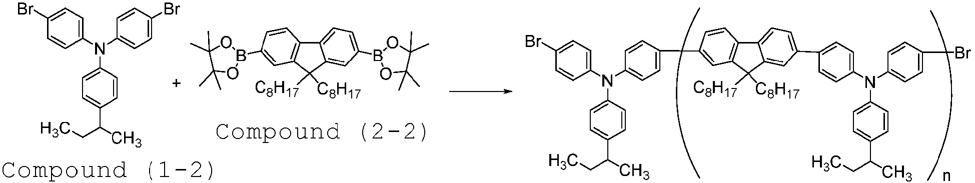

Polymer organic compound (P-1) (wherein, n represents the degree of polymerization.)

The compound (1-2) was synthesized according to a method described in International Publication WO2002/045184.

The compound (2-2) was synthesized according to a method described in US Patent No. 6169163.

A nitrogen gas atmosphere was prepared in a reaction vessel, then, the compound (1-2) (7.22 g), the compound (2-2) (9.90 g), dichlorobis[tris(2-methoxyphenyl)phosphine]palladium (4.15 mg) and toluene (131 g) were added, and the mixture was heated at 86°C. Thereafter, to this were added a 10% tetramethylammonium hydroxide aqueous solution (108 g) and tetrabutylammonium bromide (0.51 g), and the mixture was stirred at 86°C for 2 hours. The resultant reaction mixture was cooled down to room temperature, then, toluene (1096 g) was added for dilution and the liquid was separated, and water (324 g) was added and the liquid was separated. The resultant toluene layer was dropped into methanol (6853 g), then, the mixture was stirred for 1 hour, to observe a solid. The resultant solid was isolated by filtration, and dried, to obtain a polymer organic compound P-1 (10.2 g).

The polymer organic compound P-1 was analyzed by GPC, to find that the polymer organic compound P-1 had a polystyrene-equivalent number average molecular weight (Mn) of 3.5×10⁴ and a polystyrene-equivalent weight average molecular weight (Mw) of 1.05×10⁵.

The polymer organic compound P-1 was analyzed by oxygen combustion-ion chromatograph, to find that the polymer organic compound P-1 had a bromine atom content of 3700 ppm by mass. The result is shown in Table 2.

### <Comparative Example 2> Synthesis of polymer organic compound P-2

A nitrogen gas atmosphere was prepared in a reaction vessel, then, the polymer organic compound P-1 (406 mg), phenylboronic acid (7.35 mg), dichlorobis[tris(2-methoxyphenyl)phosphine]palladium (0.17 mg), tetrabutylammonium bromide (19.3 mg) and toluene (5.30 g) were added, and the mixture was heated at 80°C. Thereafter, to this was added a 10% tetramethylammonium hydroxide aqueous solution (4.25 g), and the mixture was stirred at 80°C for 1 hour. The resultant reaction mixture was cooled down to room temperature, then, toluene (20 g) was added for dilution and the liquid was separated, and water (12 g) was added and the liquid was separated. The resultant toluene layer was dropped into methanol (169 g), then, the mixture was stirred for 1 hour, to observe a solid. The resultant solid was isolated by filtration, and dried, to obtain a polymer organic compound P-2 (362 mg) .

The polymer organic compound P-2 was analyzed by oxygen combustion-ion chromatograph, to find that the polymer organic compound P-2 had a bromine atom content of 3500 ppm by mass. The result is shown in Table 2.

### <Example 2> Synthesis of polymer organic compound P-3

A nitrogen gas atmosphere was prepared in a reaction vessel, then, the polymer organic compound P-1 (407 mg), phenylboronic acid (7.37 mg), dichlorobis[tris(2-methoxyphenyl)phosphine]palladium (0.17 mg), tetrabutylammonium bromide (19.3 mg), toluene (5.30 g) and pinacol (14.0 mg) were added, and the mixture was heated at 80°C. Thereafter, to this was added a 10% tetramethylammonium hydroxide aqueous solution (4.25 g), and the mixture was stirred at 80°C for 1 hour. The resultant reaction mixture was cooled down to room temperature, then, toluene (20 g) was added for dilution and the liquid was separated, and water (12 g) was added and the liquid was separated. The resultant toluene layer was dropped into methanol (169 g), then, the mixture was stirred for 1 hour, to observe a solid. The resultant solid was isolated by filtration, and dried, to obtain a polymer organic compound P-3 (353 mg).

The polymer organic compound P-3 was analyzed by oxygen combustion-ion chromatograph, to find that the polymer organic compound P-3 had a bromine atom content of 1300 ppm by mass. The result is shown in Table 2.

### <Example 3> Synthesis of polymer organic compound P-4

A nitrogen gas atmosphere was prepared in a reaction vessel, then, the polymer organic compound P-1 (408 mg), phenylboronic acid (7.35 mg), dichlorobis[tris(2-methoxyphenyl)phosphine]palladium (0.16 mg), tetrabutylammonium bromide (19.4 mg), toluene (5.30 g) and trimethylolethane (14.4 mg) were added, and the mixture was heated at 80°C. Thereafter, to this was added a 10% tetramethylammonium hydroxide aqueous solution (4.25 g), and the mixture was stirred at 80°C for 1 hour. The resultant reaction mixture was cooled down to room temperature, then, toluene (20 g) was added for dilution and the liquid was separated, and water (12 g) was added and the liquid was separated. The resultant toluene layer was dropped into methanol (169 g), then, the mixture was stirred for 1 hour, to observe a solid. The resultant solid was isolated by filtration, and dried, to obtain a polymer organic compound P-4 (375 mg).

The polymer organic compound P-4 was analyzed by oxygen combustion-ion chromatograph, to find that the polymer organic compound P-4 had a bromine atom content of 2400 ppm by mass. The result is shown in Table 2.

**[Table 2]**

| | polymer organic compound | compound having two or more hydroxyl groups | reaction time | bromine atom content |
|---|---|---|---|---|
| Synthesis Example 1 | P-1 | - | - | 3700 ppm by mass |
| Comparative Example 2 | P-2 | none | 1 hour | 3500 ppm by mass |
| Example 2 | P-3 | pinacol | 1 hour | 1300 ppm by mass |
| Example 3 | P-4 | trimethylolethane | 1 hour | 2400 ppm by mass |

A comparison of the bromine atom content of the polymer organic compound P-2 in Comparative Example 2 with the bromine atom content of the polymer organic compound P-3 in Example 2 and the bromine atom content of the polymer organic compound P-4 in Example 3 denoted that the method for producing the present invention is excellent in the reaction rate.

### <Comparative Example 3> Synthesis of polymer organic compound P-5

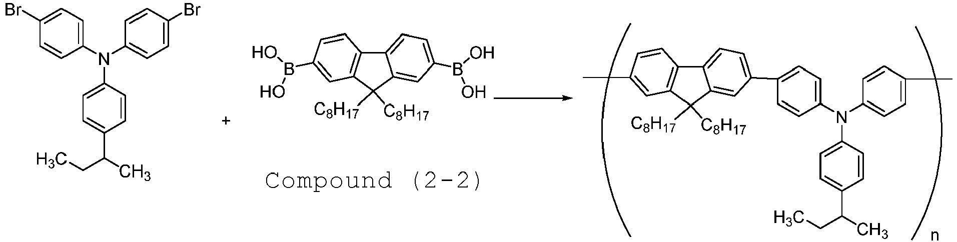

Compound (1-2) Polymer organic compound (P-5 (wherein, n represents the degree of polymerization.)

The compound (1-2) was synthesized according to a method described in International Publication WO2002/045184.

The compound (2-2) was synthesized according to a method described in "Chem. Eur. J. 2006, 12, 4351".

A nitrogen gas atmosphere was prepared in a reaction vessel, then, the compound (1-2) (2.01 g), the compound (2-2) (2.05 g), dichlorobis[tris(2-methoxyphenyl)phosphine]palladium (1.16 mg) and toluene (36 g) were added, and the mixture was heated at 86°C. Thereafter, to this were added a 10% tetramethylammonium hydroxide aqueous solution (30 g) and tetrabutylammonium bromide (0.14 g), and the mixture was stirred at 86°C for 1 hour.

The resultant reaction mixture was analyzed by GPC, to find that the polymer organic compound P-5 had a polystyrene-equivalent number average molecular weight (Mn) of 8.8×10² and a polystyrene-equivalent weight average molecular weight (Mw) of 3.0×10³. The result is shown in Table 3.

### <Example 4> Synthesis of polymer organic compound P-6 (not according to the invention)

The compound (1-2) was synthesized according to a method described in International Publication WO2002/041584.

The compound (2-2) was synthesized according to a method described in "Chem. Eur. J. 2006, 12, 4351".

A nitrogen gas atmosphere was prepared in a reaction vessel, then, the compound (1-2) (2.01 g), the compound (2-2) (2.05 g), dichlorobis[tris(2-methoxyphenyl)phosphine]palladium (1.16 mg), toluene (36 g) and pinacol (2.08 g) were added, and the mixture was heated at 86°C. Thereafter, to this were added a 10% tetramethylammonium hydroxide aqueous solution (30 g) and tetrabutylammonium bromide (0.14 g), and the mixture was stirred at 86°C for 1 hour.

The resultant reaction mixture was analyzed by GPC, to find that the polymer organic compound P-6 had a polystyrene-equivalent number average molecular weight (Mn) of 2.1×10⁴ and a polystyrene-equivalent weight average molecular weight (Mw) of 6.2×10⁴. The result is shown in Table 3.

### <Example 5> Synthesis of polymer organic compound P-7 (not according to the invention)

The compound (1-2) was synthesized according to a method described in International Publication WO2002/041584.

The compound (2-2) was synthesized according to a method described in "Chem. Eur. J. 2006, 12, 4351".

A nitrogen gas atmosphere was prepared in a reaction vessel, then, the compound (1-2) (2.01 g), the compound (2-2) (2.05 g), dichlorobis[tris(2-methoxyphenyl)phosphine]palladium (1.15 mg), toluene (36 g) and trimethylolethane (2.12 g) were added, and the mixture was heated at 86°C. Thereafter, to this were added a 10% tetramethylammonium hydroxide aqueous solution (30 g) and tetrabutylammonium bromide (0.14 g), and the mixture was stirred at 86°C for 1 hour.

The resultant reaction mixture was analyzed by GPC, to find that the polymer organic compound P-7 had a polystyrene-equivalent number average molecular weight (Mn) of 3.3×10⁴ and a polystyrene-equivalent weight average molecular weight (Mw) of 1.0×10⁵. The result is shown in Table 3.

### <Example 6> Synthesis of polymer organic compound P-8 (not according to the invention)

The compound (1-2) was synthesized according to a method described in International Publication WO2002/041584.

The compound (2-2) was synthesized according to a method described in "Chem. Eur. J. 2006, 12, 4351".

A nitrogen gas atmosphere was prepared in a reaction vessel, then, the compound (1-2) (2.01 g), the compound (2-2) (2.05 g), dichlorobis[tris(2-methoxyphenyl)phosphine]palladium (1.15 mg), toluene (36 g) and catechol (1.94 g) were added, and the mixture was heated at 86°C. Thereafter, to this were added a 10% tetramethylammonium hydroxide aqueous solution (30 g) and tetrabutylammonium bromide (0.14 g), and the mixture was stirred at 86°C for 1 hour.

The resultant reaction mixture was analyzed by GPC, to find that the polymer organic compound P-8 had a polystyrene-equivalent number average molecular weight (Mn) of 2.0×10³ and a polystyrene-equivalent weight average molecular weight (Mw) of 1.8×10⁴. The result is shown in Table 3.

**[Table 3]**

| | polymer organic compound | compound having two or more hydroxyl groups | reaction time | Mn | Mw |
|---|---|---|---|---|---|
| Comparative Example 3 | P-5 | none | 1 hour | 8.8 ×10² | 3.0 ×10³ |
| Example 4 | P-6 | pinacol | 1 hour | 2.1 ×10⁴ | 6.2 ×10⁴ |
| Example 5 | P-7 | trimethylolethane | 1 hour | 3.3 ×10⁴ | 1.0 ×10⁵ |
| Example 6 | P-8 | catechol | 1 hour | 2.0 ×10³ | 1.8 ×10⁴ |

A comparison of Mn and Mw of the polymer organic compound P-5 in Comparative Example 3 with Mn and Mw of the polymer organic compound P-6 in Example 4, Mn and Mw of the polymer organic compound P-7 in Example 5 and Mn and Mw of the polymer organic compound P-8 in Example 6 denoted that the method for producing the present invention is excellent in the reaction rate.

## Claims

1. A method for producing a polymer compound, comprising:
a step of supplying
a polymer compound (1), wherein the polymer compound (1) is a polymer compound represented by the formula (11): 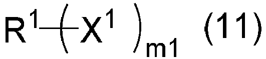 wherein
m1 represents an integer of 1 to 3,
R¹ represents a group obtained from a polymer organic compound by removing m1 hydrogen atom(s), and
X¹ represents an atom or a group selected from Group A, wherein the atom or group selected from Group A is a chlorine atom, a bromine atom, an iodine atom or a group represented by -O-S(=O)₂R^{c1}, wherein R^{c1} represents an alkyl group, a cycloalkyl group or an aryl group, the foregoing groups each optionally having a substituent,
a compound (2), wherein the compound (2) is a compound represented by the formula (12): 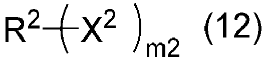 wherein
m2 represents 1,
R² represents a group obtained from an aromatic hydrocarbon, an aromatic heterocyclic compound or an aromatic amine by removing m2 hydrogen atom(s) attached directly to carbon atoms constituting the ring thereof, and
X² represents a group represented by -B(OH)₂,
a transition metal catalyst,
an organic base selected from among (CH₃)₄NOH, (C₂H₅)₄NOH, (C₃H₇)₄NOH, (C₄H₉)₄NOH, (C₅H₁₁)₄NOH, (C₁₆H₃₃)(CH₃)₃NOH, (C₈H₁₇)₃(CH₃)NOH, (C₈H₁₇)₂(C₁₀H₂₁)(CH₃)NOH, (C₈H₁₇)(C₁₀H₂₁)₂(CH₃)NOH, (C₁₀H₂₁)₃(CH₃)NOH and (C₈H₁₇)₄NOH, and
a compound having two or more hydroxyl groups represented by any one of the formula (4-1) to the formula (4-8) 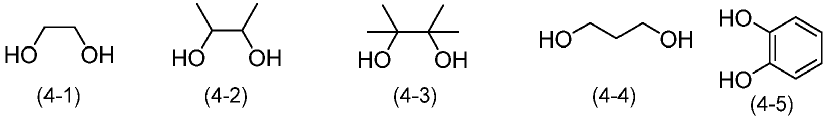 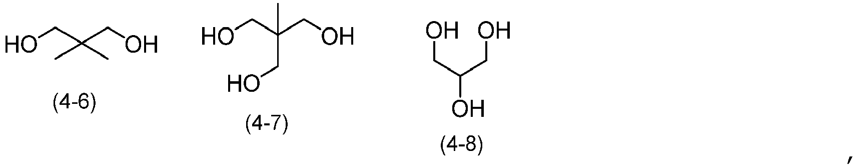 into a reaction vessel, and
a step of, in the reaction vessel, reacting the polymer compound (1) with the compound (2) in the presence of the transition metal catalyst, the organic base and the compound having two or more hydroxyl groups,
wherein the atom or group selected from group (A) in polymer compound (1) and the group represented by -B(OH)₂ in compound (2) conduct a coupling reaction to obtain a reaction product being a polymer organic compound.

2. The method for producing a polymer compound according to claim 1, wherein the at least one atom or group selected from Group A is a chlorine atom, a bromine atom, a mesylate group or a trifluoromethanesulfonate group.

3. The method for producing a polymer compound according to claim 1 or 2, wherein the transition metal catalyst is a palladium catalyst.

## Patentansprüche

1. Verfahren zur Herstellung einer Polymerverbindung, umfassend:
einen Schritt des Zuführens
einer Polymerverbindung (1), wobei die Polymerverbindung (1) eine durch die Formel (11) repräsentierte Polymerverbindung ist: 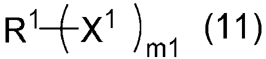 wobei
m1 eine ganze Zahl von 1 bis 3 repräsentiert,
R¹ eine aus einer organischen Polymerverbindung durch Entfernen von m1 Wasserstoffatom(en) erhaltene Gruppe repräsentiert, und
X¹ ein aus Gruppe A ausgewähltes Atom oder eine aus Gruppe A ausgewählte Gruppe repräsentiert, wobei das aus Gruppe A ausgewählte Atom oder die aus Gruppe A ausgewählte Gruppe ein Chloratom, ein Bromatom, ein lodatom oder eine durch -O-S(=O)₂R^{c1} repräsentierte Gruppe ist, wobei R^{c1} eine Alkylgruppe, eine Cycloalkylgruppe oder eine Arylgruppe repräsentiert, wobei die vorgenannten Gruppen jeweils gegebenenfalls einen Substituenten aufweisen,
einer Verbindung (2), wobei die Verbindung (2) eine durch die Formel (12) repräsentierte Verbindung ist: 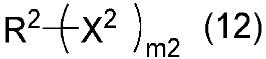 wobei
m2 1 repräsentiert,
R² eine Gruppe repräsentiert, die aus einem aromatischen Kohlenwasserstoff, einer aromatischen heterocyclischen Verbindung oder einem aromatischen Amin durch Entfernen von m2 direkt an den Ring davon bildenden Kohlenstoffatomen gebundenen Wasserstoffatom(en) erhalten wird, und
X² eine durch -B(OH)₂ repräsentierte Gruppe repräsentiert, eines Übergangsmetallkatalysators,
einer organischen Base, ausgewählt von (CH₃)₄NOH, (C₂H₅)₄NOH, (C₃H₇)₄NOH, (C₄H₉)₄NOH, (C₅H₁₁)₄NOH, (C₁₆H₃₃)(CH₃)₃NOH, (C₈H₁₇)₃(CH₃)NOH, (C₈H₁₇)₂(C₁₀H₂₁)(CH₃)NOH, (C₈H₁₇)(C₁₀H₂₁)₂(CH₃)NOH, (C₁₀H₂₁)₃(CH₃)NOH und (C₈H₁₇)₄NOH, und
einer Verbindung mit zwei oder mehr Hydroxylgruppen, repräsentiert durch eine der Formeln (4-1) bis (4-8) 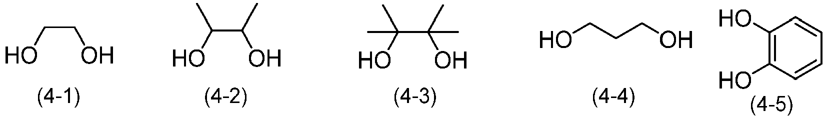 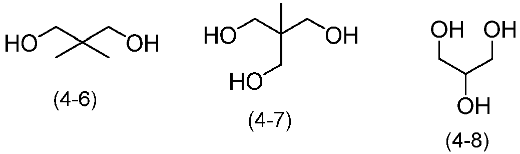 in ein Reaktionsgefäß, und
einen Schritt des Umsetzens der Polymerverbindung (1) mit der Verbindung (2) in dem Reaktionsgefäß in Gegenwart des Übergangsmetallkatalysators, der organischen Base und der Verbindung mit zwei oder mehreren Hydroxylgruppen,
wobei das aus Gruppe (A) ausgewählte Atom oder die aus Gruppe (A) ausgewählte Gruppe in der Polymerverbindung (1) und die durch -B(OH)₂ repräsentierte Gruppe in Verbindung (2) eine Kupplungsreaktion durchführen, um ein Reaktionsprodukt zu erhalten, das eine organische Polymerverbindung ist.

2. Verfahren zur Herstellung einer Polymerverbindung nach Anspruch 1, wobei das mindestens eine aus Gruppe (A) ausgewählte Atom oder die mindestens eine aus Gruppe (A) ausgewählte Gruppe ein Chloratom, ein Bromatom, eine Mesylatgruppe oder eine Trifluormethansulfonatgruppe ist.

3. Verfahren zur Herstellung einer Polymerverbindung nach Anspruch 1 oder 2, wobei der Übergangsmetallkatalysator ein Palladiumkatalysator ist.

## Revendications

1. Procédé pour la production d'un composé polymère, comprenant :
une étape d'introduction de
un composé polymère (1), où le composé polymère (1) est un composé polymère représenté par la formule (11) : 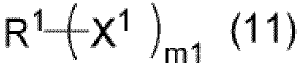 où
m1 représente un nombre entier de 1 à 3,
R¹ représente un groupe obtenu à partir d'un composé organique polymère en éliminant m1 atome(s) d'hydrogène, et
X¹ représente un atome ou un groupe choisi dans le groupe A, où l'atome ou groupe choisi dans le groupe A est un atome de chlore, un atome de brome, un atome d'iode ou un groupe représenté par -O-S(=O)₂R^{c1}, où R^{c1} représente un groupe alkyle, un groupe cycloalkyle ou un groupe aryle, les groupes précédents ayant chacun éventuellement un substituant,
un composé (2), où le composé (2) est un composé représenté par la formule (12) : 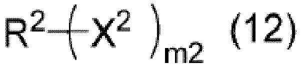 où
m2 représente 1,
R² représente un groupe obtenu à partir d'un hydrocarbure aromatique, un composé hétérocyclique aromatique ou une amine aromatique en éliminant m2 atome(s) d'hydrogène directement fixé(s) aux atomes de carbone constituant le noyau de celui-ci, et
X² représente un groupe représenté par -B(OH)₂,
un catalyseur de métal de transition,
une base organique choisie parmi (CH₃)₄NOH, (C₂H₅)₄NOH, (C₃H₇)₄NOH, (C₄H₉)₄NOH, (C₅H₁₁)₄NOH, (C₁₆H₃₃)(CH₃)₃NOH, (C₈H₁₇)₃(CH₃)NOH, (C₈H₁₇)₂(C₁₀H₂₁) (CH₃)NOH, (C₈H₁₇) (C₁₀H₂₁)₂(CH₃)NOH, (C₁₀H₂₁)₃(CH₃)NOH et (CH₈H₁₇)₄NOH, et
un composé ayant deux groupes hydroxyle ou plus représenté par l'une quelconque de la formule (4-1) à la formule (4-8) 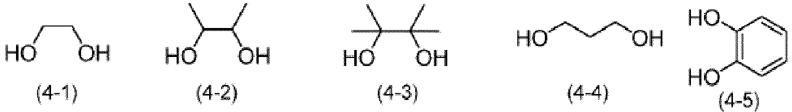 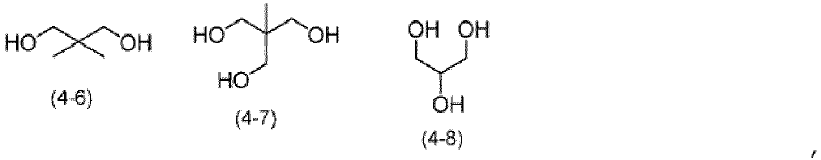 dans une cuve de réaction, et
une étape de, dans la cuve de réaction, réaction du composé polymère (1) avec le composé (2) en présence du catalyseur de métal de transition, la base organique et le composé ayant deux groupes hydroxyle ou plus,
où l'atome ou groupe choisi dans le groupe (A) dans le composé polymère (1) et le groupe représenté par -B(OH)₂ dans le composé (2) réalisent une réaction de couplage pour obtenir un produit de réaction étant un composé organique polymère.

2. Procédé de production d'un composé polymère selon la revendication 1, où le au moins un atome ou groupe choisi dans le groupe A est un atome de chlore, un atome de brome, un groupe mésylate ou un groupe trifluorométhanesulfonate.

3. Procédé pour la production d'un composé polymère selon la revendication 1 ou 2, où le catalyseur de métal de transition est un catalyseur de palladium.
